# EUROPÄISCHE PATENTSCHRIFT

(11) **EP 2 895 073 B1**
(45) Veröffentlichungstag und Bekanntmachung des Hinweises auf die Patenterteilung: **04.11.2020**
(21) Anmeldenummer: 13789701.3
(22) Anmeldetag: 04.09.2013
(51) Int. Cl.: A61B 5/20, C09J 7/22, C09J 7/38

(54) **KLEBENDER FUNKTIONSSTREIFEN ZUR TRANSKUTANEN FLUORESZENZMESSUNG**
ADHESIVE FUNCTIONAL STRIP FOR TRANSCUTANEOUS FLUORESCENCE MEASUREMENT
BANDES FONCTIONNELLES ADHÉSIVES POUR LA MESURE DE FLUORESCENCE TRANSCUTANÉE

(30) Priorität: 13.09.2012 DE 102012018076
(43) Veröffentlichungstag der Anmeldung: 22.07.2015
(73) Patentinhaber: Lohmann GmbH & Co. KG, 56567 Neuwied (DE); Gretz, Norbert, 68259 Mannheim (DE); MediBeacon, St. Louis, MO 63132 (US)
(72) Erfinder: GRETZ, Norbert, 68259 Mannheim (DE); SCHOCK-KUSCH, Daniel, 68199 Mannheim (DE); HERBERTZ, Michael, 42929 Wermelskirchen (DE); NITTENWILM, Ralf, 56203 Höhr-Grenzhausen (DE)
(74) Vertreter: Molnia, David
(86) Internationale Anmeldenummer: PCT/DE2013/000499
(87) Internationale Veröffentlichungsnummer: WO 2014/040580

(56) Entgegenhaltungen:
- EP-A1- 1 213 037
- WO-A2-2010/020673
- US-A- 5 830 136
- US-A1- 2006 229 508
- US-A1- 2007 032 721

## Beschreibung

Dem Stand der Technik entsprechende klebende Funktionsstreifen werden zum Beispiel in US2007/0032721A1 und EP1213037A1 beschrieben.

Bei der vorliegenden Erfindung handelt es sich um ein Funktionspflaster zur Messung von metabolischen Organstörungen und allgemeinen Organfunktionen von z.B. Nieren, Leber, Herz, Pankreas und Muskeln (Laktat) - insbesondere geht es um die Messung der glomerulären Filtrationsrate (GFR) - sowie ein Verfahren zur Herstellung eines solchen Funktionspflasters. Die glomeruläre Filtrationsrate gibt das von den Glomeruli (kapillaren Gefäßknäueln) beider Nieren zusammen während einer definierten Zeiteinheit gemessene Gesamtvolumen des Primärharns an, d.h. des weitgehend eiweißfreien und unkonzentrierten Harns, der bei der Durchblutung der Nieren von den Nierenkörperchen gebildet wird. Dies sind bei einem Menschen mit normalen Blutdruckwerten ca. 0,12 Liter pro Minute bzw. ca. 170 Liter pro Tag. Die GFR sinkt physiologisch mit zunehmendem Alter oder pathologisch bei Nierenerkrankungen verschiedenster Art. Die GFR ist für die Abschätzung der Nierenfunktion die wichtigste Größe. Sie wird im klinischen Alltag durch die Ermittlung der Kreatininclearance näherungsweise ermittelt. Als "Clearance" wird das Plasmavolumen bezeichnet, das pro Zeiteinheit von einer bestimmten Substanz befreit wird. Um die GFR ermitteln zu können, wird die Clearance einer Markersubstanz, also einer Substanz, die von einem Organ mit spezifischer Halbwertszeit eliminiert wird und die im Tubulussystem der Niere weder sezerniert noch rückresorbiert wird, betrachtet. Man unterscheidet dabei zwischen exogenen Markern, die dem Körper von außen durch Injektion oder Infusion zugeführt werden und endogenen, körpereigenen Markern. Innerhalb der erstgenannten Gruppe wird bevorzugt Inulin, ein physiologisch inertes Polysaccharid als Indikatorsubstanz eingesetzt. Alternativ werden heute aber auch z.B. Röntgenkontrastmittel sowie radioaktiv markierte Substanzen als exogene Markersubstanzen verwendet. Da jedoch einerseits Inulin schwer zu messen und andererseits das gesamte Verfahren mit einem recht großen Aufwand verbunden ist, erfolgt die Bestimmung der Clearance mittels Markersubstanzen in der Regel nur noch im Rahmen wissenschaftlicher Untersuchungen.

Mit für die klinische und ambulante Routinediagnostik deutlich weniger Aufwand verbunden und daher im klinischen Alltag auch bevorzugt eingesetzte Marker sind endogene Marker wie Kreatinin, ein harnpflichtiges Stoffwechselprodukt, das über den Urin und damit über die Niere ausgeschieden werden muss, oder Cystatin C, ein körpereigenes Protein.

Das erfindungsgemäße neue Funktionspflaster findet im Rahmen des Prinzips der transkutanen Fluoreszenzmessung Verwendung. Dabei wird zunächst dem Patienten eine fluoreszierende, für ein Organ spezifische Indikatorsubstanz injiziert. Eine solche Indikatorsubstanz wird mit einem Farbstoff markiert, der sich auf optischem Wege durch eine transkutane Fluoreszenzmessung nachweisen lässt. Anhand der Konzentration des Markers kann unter Berücksichtigung der jeweils gegebenen Konzentrations-Zeit-Kurve auf die Funktionstüchtigkeit des Organs zurück geschlossen werden. Die Konzentration einer solchen Substanz steigt nach der Injektion im Gewebe schnell an. Wird die Substanz dann wieder ausgeschieden, so fällt die Konzentration ab. Die Halbwertszeit dieses Abfallens ist ein Maß für die Organfunktion: Ist die Halbwertszeit kurz, so ist die Funktion des Organs gut und umgekehrt.

Das an sich bekannte Prinzip der Fluoreszenzmessung ist es, die durch Fluoreszenz entstehende Lichtstrahlung zu erfassen: Grundsätzlich ist es dabei so, dass das Anregungslicht die Probe, welche die fluoreszierende Substanz enthält, durchstrahlt. Diese fluoreszierende Substanz wandelt einen Teil des einfallenden Lichts einer bestimmten Wellenlänge in Fluoreszenzlicht einer höheren Wellenlänge um. Das so erzeugte Licht strahlt in alle Richtungen und kann mit einem Detektor z.B. senkrecht zur Achse des einfallenden Lichts gemessen werden.

Essentiell zur Erzielung objektiver und aussagekräftiger Messwerte ist es dabei, die Messung verfälschende Faktoren wie insbesondere den Einfall von Streulicht so weit wie möglich auszuschalten. Dieses Streulicht kann z.B. hervorgerufen werden durch eine Trübung infolge der Gegenwart von Feststoffen oder aber auch bei nicht-lichtdichter Anbringung des Sensors auf der Haut durch den dann gegebenen seitlichen Lichteinfall.

Ansonsten liegt der wesentliche Unterschied bei solchen Tests im Einsatz unterschiedlicher Marker, die bei unterschiedlichen Wellenlängen fluoreszieren und wellenlängenbedingt unterschiedlich tief eindringen.

Neben der Heranziehung unterschiedlicher Markersubstanzen, auf die an dieser Stelle nicht detaillierter eingegangen werden soll, wurden und werden im Laufe der Zeit immer neue Messverfahren getest und auch in der Patentliteratur beansprucht. In älteren Schutzrechten wie z.B. DE 32 24 641 A1 wird die GFR-Bestimmung mittels HPLC beansprucht, in neueren mittels Magnetresonanztherapie (DE 10 2008 050 347 B4), CT (US 7,813,536 B2) oder Messung der UV-Absorbanz mindestens einer harnpflichtigen Substanz im Dialysatausfluss oder im Blutfluss der Blutbehandlungseinheit (DE 10 2010 047 215 A1 und DE 10 2011 012 674 B3). EP 0 421 697 A2 beschreibt - wie es auch in ähnlicher Form in einigen weiteren Schutzrechten geschieht - ein bildgebendes Magnetresonanzverfahren zur Bestimmung der GFR, EP 1 111 387 B1 behandelt immunologische Analysenmethoden. EP 1 632 320 B1 thematisiert in diesem Zusammenhang allgemein immunanalytische Techniken, chromatographische Techniken, Massenspektrometrie, Abbildungstechniken und radioaktive Zähltechniken und Kern der US 5,989,298 A ist eine kapillare Elektrophorese-Methode. US 2003/0019115 A1 schließlich beansprucht eine nutzerfreundliche, tragbare, elektronische Vorrichtung, die in kürzester Zeit Daten eines nicht näher spezifizierten Messverfahrens zur patientenspezischen GFR errechnet.

Im Zuge einer angestrebten weiteren Vereinfachung der Untersuchung von Organfunktionen gibt es nun Verfahren, mittels neu entwickelter Geräte die jeweiligen Organfunktionen und hier insbesondere die GFR, aber auch die Funktionen von Leber, Herz, Pankreas oder Stoffwechselfunktionen transkutan und nicht durch Blutuntersuchungen zu bestimmen.

Dabei werden elektronische Sensoren über einen definierten Zeitraum auf der Haut des Probanden befestigt. Mittels einer selbstklebenden Kopplungsschicht wird der Sensor möglichst druckfrei und ohne die Mikrozirkulation der Haut zu stören (vgl. dazu Lim et al.: "Probe pressure effects on human skin diffuse reflectance and fluorescence spectroscopy measurements" in: Journal of Biomedical Optics 16(1), January 2011) in einer definierten Position über einen definierten Zeitraum auf der Haut gehalten und nach Beendigung der Untersuchung auch wieder rückstandsfrei sowie ohne Irritationen oder Verletzung von Hautzellgewebe von der Haut entfernt. Nächstliegender Stand der Technik ist hier die WO 2010/020673 A2, in der ein Sensorpflaster zur transkutanen Messung einer Organfunktion, insbesondere der Nierenfunktion vorgeschlagen wird, wobei die Körperoberfläche mit mindestens einem Abfrage- oder Erregerlicht bestrahlt wird und ein Detektor das aus der Richtung der Körperoberfläche eingestrahlte Antwortlicht erfasst. Das in dieser WO beschriebene Pflaster ist bevorzugt so aufgebaut, dass die Strahlenquelle integraler Bestandteil des Pflasters ist, Aussagen über Anforderungen an das Pflaster sowie dessen Aufbau werden in der genannten WO-Anmeldung lediglich in sehr verallgemeinerter Form gemacht. Dabei spielt gerade dieser Punkt eine wesentliche Rolle, denn wie bereits erwähnt besteht eine wesentliche Anforderung an das Pflaster darin, seine Geometrie und Gesamtkonstruktion so auszulegen, dass Streulicht absorbiert und die Fluoreszenzmessung nicht gestört wird. Dies setzt voraus, dass der Sensorkopf lichtdicht auf der Haut angebracht werden muss, um das Eindringen von Streulicht zu verhindern, auf der anderen Seite aber darf er auch nicht zu fest aufsitzen, da ansonsten die Mikrozirkulation in der Haut gestört wird. Dadurch wiederum würden die Halbwertszeiten verlängert und die gemessenen Werte verfälscht.

Gelöst wurde diese Anforderung durch ein mehrlagig aufgebautes Klebepflaster, bei dem anders als in WO 2010/020673 A2 die Strahlenquelle nicht innerhalb eines schichtartigen Aufbaus ein integrativer Bestandteil des Pflasters ist, wobei der Begriff "Pflaster" hier alle möglichen Arten eines klebend ausgerüsteten filmförmigen Gegenstands umfasst: Das gesamte Sensorelement ist vom eigentlichen Pflaster abgekoppelt und wird als gesondertes Teil auf das Pflaster aufgebracht. Damit können also sowohl das Pflaster als auch der Sensor mehrfach Verwendung finden oder aber auch als nur einmal verwendbarer und dann zu entsorgender Artikel gebraucht werden. Der spezifische mehrlagige Aufbau des Pflasters verhindert einerseits, dass das Streulicht, welches teilweise auch seitlich in der Haut "wandern" kann, bis zur Photodiode kommt, da in der Nähe eingestrahltes Licht absorbiert wird und andererseits, dass Hautteile nach dem Aufsetzen des Sensorkopfes von unten in den Sensorkopf eindringen. Im letzteren Falle würde die Durchblutung im eingedrungenen und gleichzeitig aber auch für die Messung benutzten Gewebe verlangsamt. Hierdurch würde auch die Ausscheidung des Markers aus dem so eingeklemmten Gewebe verlangsamt und der gemessene Kurvenverlauf wäre ebenfalls langsamer, das Messergebnis mithin verfälscht. Eines solche Störung der Mikrozirkulation wird durch die unterste, durchgängige und transparente Schicht des Pflasters verhindert. Das erfindungsgemäße Klebepflaster stellt eine einfach zu handhabende, gleichzeitig aber wenig aufwändige, schnelle und zuverlässige Methode zur Messung von Organfunktionen und hier insbesondere der Nierenfunktion dar.

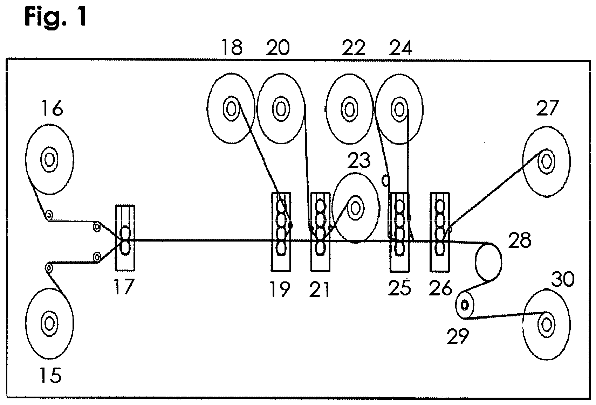

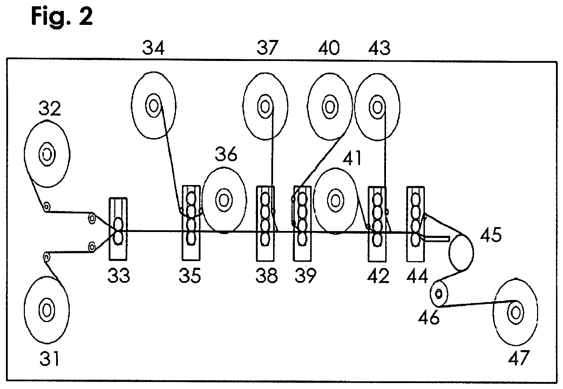

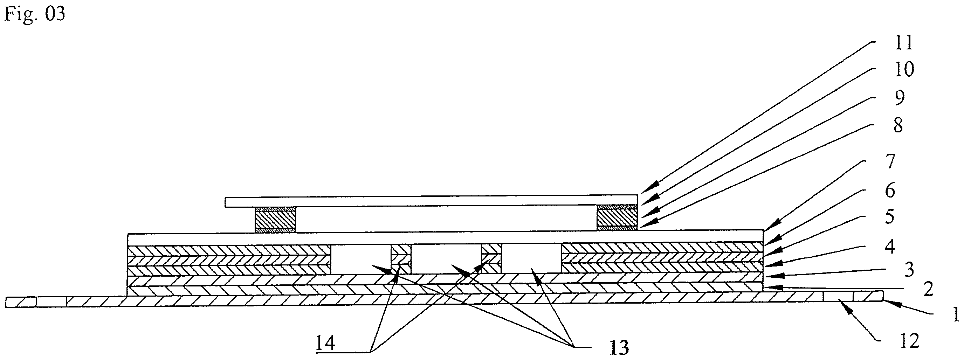

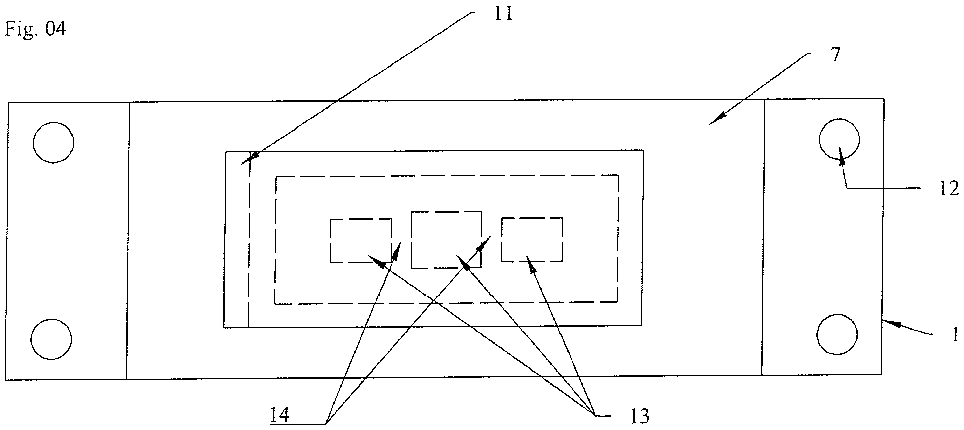

Der sandwichartige Aufbau des bevorzugt als Formstanzteil vorliegenden Pflasters aus mehreren Schichten wird anhand der Fig. 3 im Folgenden näher erläutert. Dieses Formstanzteil weist zunächst auf der der Haut zugewandten Seite eine die Klebefläche zur Haut hin abdeckende Folie als Release Liner auf (1). Bei diesem Liner wird wie bei allen im sandwichartigen Aufbau eingesetzten Linern eine abgestuft silikonisierte Polyesterfolie bevorzugt. Die Abstufung, d.h. das Verhältnis der Release-Werte der beiden Linerseiten sollte dabei zwischen 1:2 und 1:10 liegen. Die Abstufung ist dabei in Abhängigkeit von der Klebkraft des Klebstoffs angepasst an die Erfordernisse der sicheren Fixierung des Stanzteils auch beim Aufwickeln auf Rollen. Die Folie hat allerdings noch eine weitere Funktion: Sie weist einen beidseitig jeweils über die Klebefläche hinausgehenden Überstand mit Positionslöchern (12) auf. Diese Positionslöcher dienen bei der Herstellung des gesamten Funktionsteils aus Sensor und Pflaster der passgenauen Befestigung des Sensors auf dem Pflaster. Der Liner deckt bis zu deren Gebrauch eine biokompatible, für den medizinischen Gebrauch auf der Haut zugelassene Klebstoffschicht (2) wahlweise auf Basis Acrylat, Silikon oder Kautschuk ab. Auf der der Haut abgewandten Seite dieser Klebstoffschicht ist eine transparente Polyesterfolie (3) aufgebracht. Statt zweier separater Komponenten wie einem trägerlosen Transferhaftklebeband (2) und der Polyesterfolie (3) wäre hier allerdings auch ein einseitig klebendes Haftklebeband mit einem transparenten Trägermaterial und einer biokompatiblen Klebeschicht einsetzbar, wodurch bei der Herstellung ein Kaschiervorgang entfallen könnte und damit auch Kosten gesenkt würden.

Die transparente Polyesterfolie ist verklebt mit einem farblich dunkel modifizierten, bevorzugt schwarzen Klebstoff (4). Aus Stabilitätsgründen im Rahmen des Stanzvorgangs bei der Herstellung des Pflasters wird hier ein doppelseitig haftklebend ausgerüstetes Band (4 - 6) mit einem dunklen/schwarzen Klebstoff (4, 6) am ehesten in Frage kommen, doch auch der Einsatz eines trägerlosen Transferbands wäre durchaus möglich. Diese dunkle(n) Klebstofflage(n) enthält/enthalten zumindest eine Aussparung, oberhalb derer beim Zusammenbau des kompletten Funktionsteils aus Pflaster und Sensor der Sensorkopf positioniert wird. Diese Aussparung, hergestellt z.B. mittels eines Stanzvorgangs, stellt ein optisches Fenster dar, welches dem Sensor mit seinen LEDs und Photodioden einen ungestörten Einblick auf die Haut ermöglicht.

Die der ersten Polyesterfolie (3) abgewandte Klebstoffschicht (6) wird von einer weiteren transparenten, die zumindest eine Aussparung überdeckenden und damit auch das Gesamtsystem stabilisierenden Polyesterfolie (7) abgedeckt, auf der abschließend ein umlaufender Rahmen (8 - 10) z.B. aus einem entsprechend den Erfordernissen gestanzten, doppelseitig haftklebend ausgerüsteten und vor Gebrauch von einem Liner mit einer überstehenden Anfasslasche abgedeckten Schaumträgermaterial (11) klebend angebracht wird. Dieser Rahmen (8 - 10) dient dazu, den Einfall von die Messergebnisse verfälschendem Streulicht zu verhindern. Im besonders bevorzugten Fall ist dieser Rahmen integrativer Bestandteil des Funktionspflasters, denkbar wäre aber auch ein vom Funktionspflaster separater Dichtungsrahmen, der dann als doppelseitig oder auch nur als einseitig klebendes separates Bauteil vorliegen würde und vor Aufbringung des Sensors an der passenden Stelle auf dem Funktionspflaster angebracht würde. In einer weiteren Anwendungsform könnte der Dichtungsrahmen auch dauerhaft an dem Sensor befestigt sein, er würde in einem solchen Falle dann im Prinzip gemeinsam mit dem Sensor auf das Funktionspflaster aufgeklebt.

### Beispiel für den Aufbau der Funktionspflaster:

In den erfolgreichen Versuchen mit diesem Aufbau kam als Kopplungsschicht zur Haut hin der für medizinische Anwendungen geeignete Transferkleber "DuploMED VP 8171" zum Einsatz, ein wie auch die in der Folge hier genannten Klebebänder von der Firma "Lohmann GmbH & Co. KG" hergestelltes und vertriebenes Klebeband, in diesem Falle ein 50µm dickes Transferklebeband (2) mit Silikonpapierabdeckung. Der Haftklebstoff ist ein biokompatibler lösemittelbasierter Polyacrylat-Klebstoff, der aus zwei Komponenten besteht: der Polyacrylat- und einer Vernetzerlösung. Er erfüllt die Anforderungen der DIN EN 10993-1 und wird vorwiegend eingesetzt in medizinischen Pflastern, Folien und sonstigen klebenden Verbandstoffen. Der Haftklebstoff wird an einer für die medizinische Fertigung zugelassenen Beschichtungsanlage im Walzenauftragsverfahren in einer Dicke von 50 µm auf ein silikoniertes 90g/m² Kraftpapier beschichtet, getrocknet und vernetzt. Die ursprüngliche Silikonpapierabdeckung wird für den Funktionspflaster-Herstellvorgang durch eine 50 µm dicke, silikonisierte Polyethylenterephthalat-Releasefolie (1) ersetzt.

Die transparente Klebstoffschicht (2) wirkt in dem hier vorgesehenen Anwendungsfalle auch als Diffusor und erlaubt somit beim Sensor die Nutzung sehr kleiner punktförmiger LEDs. Die Verwendung solcher LEDs ist im Rahmen der Miniaturisierung des Sensorkopfes erforderlich. Grundsätzlich aber kann auch jeder andere Klebstoff, der diese geschilderten Bedingungen erfüllt, hier zum Einsatz kommen.

Die mit diesem Transferkleber auf der der Haut abgewandten Seite verklebte transparente Polyesterfolie (3) dient zur Dimensionsstabilisierung des Funktionsstanzteilrahmens und als Barriere bzw. Spacer zwischen der medizinischen (Haut) und technischen (Sensor) Seite der Kopplungsschicht. Zum Sensor hin kommt mit "DuploCOLL® VP 6899" ein 0,12 mm dickes, doppelseitiges Haftklebeband mit einem 12µm Polyesterträger (5) und einem russpigmentierten, harzmodifizierten Acrylatklebstoff auf Lösemittelbasis (4, 6) zum Einsatz. Die Rußpigmentierung verleiht dem Klebstoff eine lichtabsorbierende Funktion. Grundsätzlich sind aber auch hier wieder alle Klebstoffsysteme geeignet, die die Bedingungen einer sehr guten Verklebung mit der transparenten Polyesterfolie (3) auf der einen und dem Sensorgehäuse auf der anderen Seite erfüllen und gleichzeitig lichtabsorbierend ausgerüstet sind.

Aus den beiden schwarz pigmentierten Klebstoffschichten (4-6) sowie dem entsprechenden Polyesterträgermaterial (5) wird im Fertigungsprozess zumindest ein "Fenster" (13) ausgestanzt oder durch geeignete Maßnahmen in der Klebstoffschicht ausgespart, in einer bevorzugten Ausführung werden mehrere nebeneinander liegende "Fenster" (13) ausgestanzt bzw. ausgespart und zwar in der Weise, dass die Fenster durch schmale Stege (14) voneinander getrennt sind, damit pro Fenster auch nur eine Lichtquelle einstrahlt. Oberhalb dieser Fenster wird vor Gebrauch der Sensorkopf mit LEDs und Photodiode in der Weise angebracht, dass durch die schwarzen Stege zwischen den LEDs und der Photodiode das direkte Eindringen von Licht aus den LEDs in die Photodiode verhindert wird. Die der Haut abgewandte Seite des schwarzen Klebebands wird von einer transparenten Polyesterfolie (7) abgedeckt. Diese Folie (7) hat neben der Abdeckung auch die Funktion, die an der Ausstanzung bzw. den Ausstanzungen (13) entstandenen Ränder des Pflasters zu stabilisieren.

Zur Verhinderung eines eventuellen seitlich einfallenden und deshalb störenden Streulichts wird die der Haut abgewandte und von der transparenten Folie abgedeckte Seite des schwarzen Klebebandes abschließend noch mit einer das Fenster bzw. die Fenster umlaufenden Rahmendichtung (8 - 10) versehen. Diese Dichtung besteht bevorzugt aus einem doppelseitigen Haftklebeband mit einem Schaumträger (9), im vorliegenden Beispiel ist dies "DuploCOLL ® 9042", ein Reinacrylathaftklebstoff (8, 10), beschichtet auf beide Seiten eines Polyethylen-Vinylacetat-Copolymerschaumes (9). Infolge des Schaumstoffträgers weist diese Dichtung eine gewisse Elastizität und Flexibilität auf, die ein Reagieren auf und eine Anpassung an eventuelle Bewegungen des Sensorteils ermöglichen.

Anhand von im "Laser Prototyping"- Verfahren hergestellten kleineren Stückzahlen dieser Konstruktion konnten dann reproduzierbare Messergebnisse in einem transkutanen Verfahren erzielt werden - Bei diesem Herstellverfahren wurden in einem diskontinuierlichen "Sheet-to-Sheet"- Prozess einzelne Bögen manuell mit Hilfe eines Flachbettlasers so bearbeitet, dass schließlich Prototypen des Funktionspflasters in geringer Stückzahl hergestellt werden konnten.

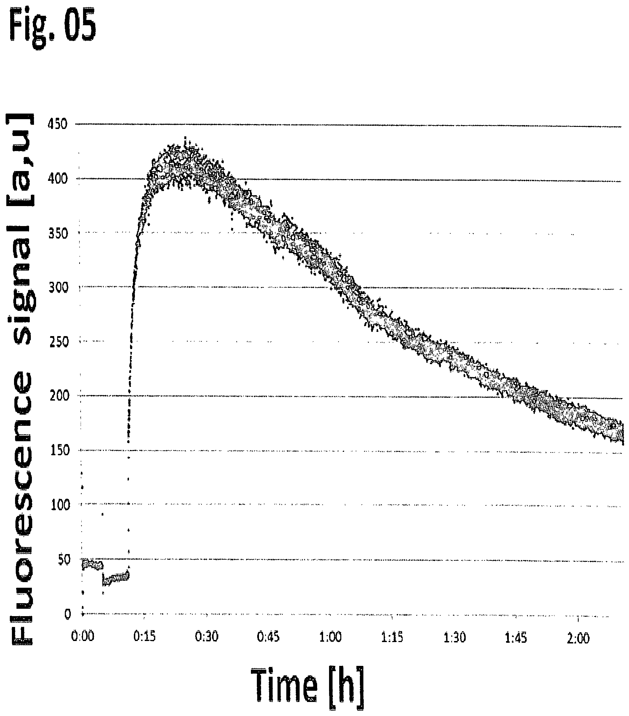

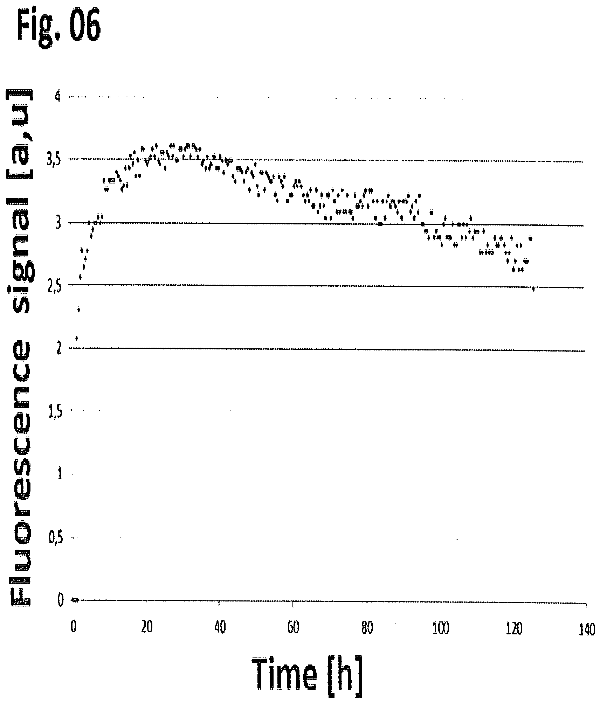

Auf Grund von ersten Versuchen mit diesen Prototypen konnte dann nachgewiesen werden (vgl. dazu Fig. 5 und 6), dass das Streulicht absorbiert und die Mikrozirkulation der Haut nicht gestört wurde. Fig. 5 zeigt die Ausscheidungskurve bei Einsatz des erfindungsgemäßen Funktionspflasters und Fig. 6 den Kurvenverlauf bei andersartiger Befestigung des Sensors auf der Haut: Die Kurve in Fig. 6 verläuft in atypisch abgeflachter Form, ein deutliches Zeichen dafür, dass der Sensor offenbar zur Verhinderung des Einfalls von Streulicht mit erhöhtem Druck befestigt wurde, was wiederum zu einer Verformung der Hautschichten und damit zu einem unsachgemäßen und verfälschten Messergebnis führt. Das erfindungsgemäße Funktionspflaster ließ sich nach Beendigung der Messung rückstandsfrei und ohne Verletzungen zu hinterlassen wieder ablösen.

### Herstellweise der Funktionspflaster:

Die maschinelle Herstellung der Funktionspflasterteile schließlich erfolgt bevorzugt in mehreren kontinuierlichen "Roll-to-Roll"- bzw. "Roll-to-Sheet" - Kaschier- und Stanzprozessen, d.h. die einzelnen Bestandteile des Funktionspflasters sind auf Rollen gelagert und werden im Stanzprozess sukzessive kontinuierlich an den dafür vorgesehenen Stellen innerhalb des Prozesses abgerollt und entweder wiederum zu Rollen oder aber zu Bogen mit den eigentlichen Funktionspflastern verarbeitet.

In einem ersten Schritt wird der Dichtungsrahmen des Funktionspflasters hergestellt. Dazu wird zunächst das mit einer HDPE-Folie abgedeckte doppelseitige Klebeband "Duplocoll 9042" mit einem Schaumträger (16) in einer Kaschierstation (17) auf eine schwach haftklebend ausgerüstete Prozessfolie (15), die dann im weiteren Prozess als Stanzunterlage fungiert, aufgebracht. Im nächsten Schritt werden die Konturen des Dichtungsrahmens ausgestanzt (19), das Stanzgitter wird separiert und aufgerollt (18). Im folgenden Schritt werden die inneren Stanzabdeckungen mit einem Nockenwerkzeug und mit Hilfe eines Abzugsklebers (20) abgezogen (21). Bei diesem Abzugskleber handelt es sich um ein handelsübliches Klebeband, welches dergestalt getaktet auf den Verbund zuläuft, dass es lediglich immer nur partiell in genau definierten bestimmten Abständen in Kontakt mit dem Verbund tritt und in dieser Weise auch nur die zuvor aus dem Verbund gelösten Teile daraus entfernt. Auch diese aus dem weiteren Herstellungsprozess entfernten inneren Stanzabdeckungen werden dann aufgerollt (23). Im nachfolgenden Schritt wiederholt sich diese Vorgehensweise im Prinzip: Wiederum wird den noch im Fertigungsprozess verbliebenen ausgestanzten Teilen Abzugskleber (22) zukaschiert (25), der die Stanzabdeckung und die Kleberinnenteile abzieht und aufrollt (24). An der nächsten Station wird die Anfasslasche gestanzt (26) und nachfolgend das Linergitter abgezogen und aufgerollt (27). Über eine Transportwalze (28) und eine Zugwalze zur Transportwalze (29) wird der fertige Dichtungsrahmen schließlich aufgewickelt (30).

Unabhängig von der Herstellung des Dichtungsrahmens wird parallel dazu in einem zweiten Stanzprozess das für die Anwendung auf der menschlichen Haut geeignete doppelseitig haftklebende Band "Duplocoll 8171" (31) mit einer nicht silikonisierten 50µm Polyethylenterephthalatfolie (32) zusammen kaschiert (33). Gleichzeitig wird in einem gegenläufigen Prozess die Innenkontur des mit einer Stanzabdeckung abgedeckten doppelseitig mit schwarz pigmentierten Klebstoffschichten haftklebend ausgerüsteten Bandes "Duplocoll VP 6899" (36) ausgestanzt (35), d.h. es wird das "Fenster" bzw. bei mehreren werden die "Fenster" ausgestanzt.

Anschließend wird die Stanzabdeckung mit den Innenteilen abgezogen (34), während die für den weiteren Fertigungsprozess benötigten ausgestanzten Teile des "Duplocoll VP 6899" jetzt mit "Duplocoll 8171" (31) sowie der Polethylenterephthalatfolie (32) zusammen kaschiert werden (38), gleichzeitig wird der Originalliner des "Duplocoll VP 6899" abgezogen (37).

An der nächsten Kaschierstation (39) wird die 12 µm dicke PETP-Folie (40) zulaufen lassen. Daran anschließend werden die bereits im oben beschriebenen ersten Fertigungsschritt ausgestanzten Dichtungsrahmen (30) von dem Abwickler (41) hinzu kaschiert (42), bevor von dem gesamten System die schwach haftklebend ausgerüstete Prozessfolie (15) abgezogen wird (43). Damit liegt nun der für die Funktionsweise des Systems notwendige Schichtenaufbau vor, aus dem an einer weiteren Stanzstation (44) die endgültige Form der erfindungsgemäßen Funktionspflaster ausgestanzt wird. Die fertigen Teile werden nun separiert, das verbleibende Stanzgitter wird über eine Transportwalze (45) und eine Zugwalze zur Transportwalze (46) aufgewickelt (47).

### Verwendung der erfindungsgemäßen Funktionspflaster:

Die erfindungsgemäßen Funktionspflaster können in zweifacher Form Verwendung finden: Zum einen können Pflaster und Sensorteil als untrennbare Einheit vorliegen, die dann auch nach Gebrauch gemeinsam entsorgt werden oder sie können getrennt und unabhängig voneinander vorliegen und müssen erst vor Gebrauch zusammengefügt werden. Im letzteren Falle kann z.B. ein Funktionspflaster auch über längere Zeit auf der Haut verbleiben und nacheinander mit verschiedenen Sensoren bestückt werden.

### Liste der Bezugszeichen:

- 1 =: PETP-Folie silikonisiert, mit Überstand und Positionslöchern
- 2 =: Klebstoffschicht
- 3 =: PETP-Folie nicht silikonisiert,
- 4 =: Klebstoffschicht
- 5 =: Polyester-Folie, nicht silikonisert,
- 6 =: Klebstoffschicht
- 7 =: PETP-Folie, nicht silikonisiert,
- 8 =: Klebstoffschicht
- 9 =: Schaumschicht
- 10 =: Klebstoffschicht
- 11 =: Polyesterfolie silikonisiert mit Anfasslasche
- 12 =: Positionslöcher
- 13 =: Ausstanzung
- 14 =: Stege
- 15 =: Zulauf schwach haftende Prozessfolie
- 16 =: Zulauf "DuploCOLL ® 9042"
- 17 =: Kaschierstation
- 18 =: Aufrollung Stanzgitter
- 19 =: Stanze Kontur
- 20 =: Abzugskleberrolle
- 21 =: Kaschierung Abzugskleber auf innere Stanzabdeckungen
- 22 =: Abzugskleberrolle
- 23 =: Aufrollung Abzugskleber mit inneren Stanzabdeckungen
- 24 =: Aufrollung Abzugskleber mit äußeren Stanzabdeckungen und Kleberinnenteilen
- 25 =: Kaschierung Abzugskleber auf äußere Stanzabdeckungen und Kleberinnenteile
- 26 =: Stanze Anfasslasche
- 27 =: Aufrollung Linergitter
- 28 =: Transportwalze
- 29 =: Zugwalze zur Transportwalze
- 30 =: Produktaufwickler "Dichtungsrahmen"
- 31 =: Zulauf "DuploCOLL ® 8171"
- 32 =: Zulauf PETP-Folie, nicht silikonisiert
- 33 =: Kaschierstation
- 34 =: Aufrollung Stanzabdeckung mit Innenteilen
- 35 =: Stanze Innenkontur
- 36 =: Zulauf "DuploCOLL ® VP 6899" abgedeckt mit Stanzabdeckung
- 37 =: Aufrollung Original-Liner "DuploCOLL® VP 6899"
- 38 =: Kaschierstation
- 39 =: Kaschierstation
- 40 =: Zulauf PETP-Folie, nicht silikonisiert
- 41 =: Zulauf vorgefertigte Stanzteile "Dichtungsrahmen"
- 42 =: Kaschierstation
- 43 =: Aufrollung schwach haftklebende Prozessfolie
- 44 =: Stanze
- 45 =: Transportwalze
- 46 =: Zugwalze zur Transportwalze
- 47 =: Aufrollung Stanzgitter

## Patentansprüche

1. Klebender Funktionsstreifen zur transkutanen Fluoreszenzmessung von allgemeinen Organfunktionen oder metabolischen Organstörungen mit Hilfe eines auf dem Streifen befestigten Sensorkopfes, der mit LED-Licht und einer Photodiode ausgestattet ist, wobei der Streifen im wesentlichen aus einem Laminat folgender Schichten besteht:
- Trägerloser transparenter Transferklebstreifen oder doppelseitig haftklebend ausgerüstetes und für Anwendungen auf der Haut geeignetes transparentes Klebeband (2)
- Transparente Folie (3)
- Mindestens eine lichtabsorbierende Haftklebeschicht (4-6) mit mindestens einer Aussparung (13), durch die der direkte Lichtkontakt sowohl des mit LED-Licht ausgerüsteten Sensorkopfes als auch der Photodiode mit der Haut ermöglicht wird,
- Stabilisierende transparente Abdeckung (7), worauf, auf der der Haut abgewandten Seite des Funktionsstreifens, ein umlaufender, vier Dichtungsstreifen (8-11) umfassender Dichtungsrahmen angebracht ist, der ein integrativer Bestandteil des Funktionspflasters ist.

2. Klebender Funktionsstreifen gemäß Anspruch 1, **dadurch gekennzeichnet, dass** der Transferklebestreifen bzw. das für Anwendungen auf der Haut geeignete transparente Klebeband (2) einen Klebstoff wahlweise auf Basis Acrylat, Silikon oder Kautschuk umfasst.

3. Klebender Funktionsstreifen gemäß Anspruch 1, **dadurch gekennzeichnet, dass** die mindestens eine lichtabsorbierende Haftklebeschicht (4) aus einem rußpigmentierten und infolgedessen schwarzen Klebstoff auf Basis Acrylat, Silikon oder Kautschuk besteht.

4. Klebender Funktionsstreifen gemäß Anspruch 1 oder Anspruch 2, **dadurch gekennzeichnet, dass** die lichtabsorbierende Haftklebeschicht (4-6) in Form eines trägerbasierten, doppelseitig mit einem rußpigmentierten und infolgedessen schwarzen Klebstoff auf Basis Acrylat, Silikon oder Kautschuk versehenen Klebebands (5) vorliegt.

5. Klebender Funktionsstreifen gemäß Anspruch 1, **dadurch gekennzeichnet, dass** das doppelseitige lichtabsorbierende Klebeband mehrere nebeneinander liegende, durch lichtundurchlässige Stege (14) voneinander getrennte fensterförmige Aussparungen (13) enthält, durch die ein direkter Lichtkontakt sowohl des mit LED-Licht ausgerüsteten Sensorkopfes als auch der Photodiode mit der Haut ermöglicht wird.

6. Klebender Funktionsstreifen gemäß Anspruch 1, **dadurch gekennzeichnet, dass** die Dichtungsstreifen (8-11) aus einem beidseitig mit einem Klebstoff (8,10) auf Basis Acrylat, Silikon oder Kautschuk beschichteten Schaum (9) bestehen.

7. Verfahren zur Herstellung klebender Funktionsstreifen gemäß einem der vorhergehenden Ansprüche, **dadurch gekennzeichnet, dass** die Aussparung/en (13) durch einen Stanzprozess hergestellt wird/werden.

8. Verfahren zur Herstellung klebender Funktionsstreifen gemäß einem der vorhergehenden Ansprüche 1 bis 6, **dadurch gekennzeichnet, dass** er in einer Abfolge von Kaschier- und Stanzvorgängen hergestellt wird.

9. Verwendung eines klebenden Funktionsstreifens gemäß einem der vorhergehenden Ansprüche 1 bis 6, **dadurch gekennzeichnet, dass** er wahlweise mehrfach wieder verwendbar oder nach einmaligem Gebrauch zu entsorgen ist.

10. Verwendung eines klebenden Funktionsstreifens gemäß einem der vorhergehenden Ansprüche 1 bis 6, **dadurch gekennzeichnet, dass** er zur transkutanen Messung der glomerulären Filtrationsrate (GFR) verwendet wird.

11. Verwendung eines klebenden Funktionsstreifens gemäß einem der vorhergehenden Ansprüche 1 bis 6, **dadurch gekennzeichnet, dass** er wahlweise zur transkutanen Messung von Leber-, Herz- oder Pankrasfunktionen oder auch zur Messung von Laktat- oder Glucosewerten verwendet wird.

## Claims

1. Adhesive functional strip for transcutaneous fluorescence measurement of general organ functions or metabolic organ disorders with the aid of a sensor head which is fastened to the strip and provided with LED light and a photodiode, wherein the strip consists substantially of a laminate of the following layers:
- backing-less transparent transfer adhesive strip or transparent adhesive tape (2) provided on both sides with an adhesive coating suitable for applications to the skin,
- transparent film (3),
- at least one light-absorbing pressure-sensitive adhesive layer (4-6) with at least one recess (13), through which a direct light contact with the skin of both the sensor head equipped with LED light and the photodiode is made possible,
- stabilising transparent cover (7) on which a circumferential sealing frame comprising four sealing strips (8-11) is applied on the side remote from the skin, the sealing frame being an integral component of the functional patch.

2. Adhesive functional strip according to claim 1, **characterised in that** the transfer adhesive strip or the transparent adhesive tape (2) which is suitable for applications to the skin comprises an adhesive on the basis of either acrylate, silicone, or rubber.

3. Adhesive functional strip according to claim 1, **characterised in that** the at least one light-absorbing pressure-sensitive adhesive layer (4) consists of a soot-pigmented and consequently black adhesive on the basis of acrylate, silicone or rubber.

4. Adhesive functional strip according to claim 1 or claim 2, **characterised in that** the light-absorbing pressure-sensitive adhesive layer (4-6) is present in the form of a backing-based adhesive tape (5) provided on both sides with a soot-pigmented and consequently black adhesive on the basis of acrylate, silicone or rubber.

5. Adhesive functional strip according to claim 1, **characterised in that** the double-sided light-absorbing adhesive tape contains a plurality of window-shaped recesses (13) located next to one another and separated from one another by light-impermeable webs (14), through which recesses (13) the direct light contact both of the sensor head equipped with LED light and also of the photodiode with the skin is made possible.

6. Adhesive functional strip according to claim 1, **characterised in that** the sealing strips (8-11) consist of a foam (9) coated on both sides with an adhesive (8, 10) on the basis of acrylate, silicone or rubber.

7. Method for producing an adhesive functional strip according to one of the preceding claims, **characterised in that** the recess(es) (13) is/are produced by a punching process.

8. Method for producing an adhesive functional strip according to one of the preceding claims 1 to 6, **characterised in that** it is produced in a sequence of lamination and punching operations.

9. Use of an adhesive functional strip according to one of the preceding claims 1 to 6, **characterised in that** it is either capable of being re-used several times or is to be disposed of after a single use.

10. Use of an adhesive functional strip according to one of the preceding claims 1 to 6, **characterised in that** it is used for transcutaneous measurement of the glomerular filtration rate (GFR).

11. Use of an adhesive functional strip according to one of the preceding claims 1 to 6, **characterised in that** it is used either for transcutaneous measurement of liver, heart or pancreas functions or also for measuring lactate or glucose values.

## Revendications

1. Bandes fonctionnelles adhésives pour la mesure de fluorescence transcutanée des fonctions générales des organes ou des troubles métaboliques des organes au moyen d'une tête de capteur montée sur la bande,
et équipée de lampe LED et d'une photodiode, la bande étant constituée essentiellement d'un stratifié avec les couches suivantes:
- Bande adhésive de transfert transparente sans porteur ou bande adhésive transparente autocollante double face adaptée aux applications cutanées (2)
- Filme transparent (3)
- Au moins une couche adhésive sensible à la lumière (4-6) avec au moins une encoche (13), mettant les sources lumineuses, c'est-à-dire la tête du capteur avec lampe LED et la photodiode, directement en contact avec la peau,
- Un cache transparent (7) avec un cadre d'étanchéité périphérique faisant partie intégrante du sparadrap fonctionnel, situé sur le côté des bandes fonctionnelles tourné vers la peau et formé de quatre bandes d'étanchéité (8-11).

2. Bandes fonctionnelles adhésive
caractérisées selon la revendication 1 en ce que la bande adhésive de transfert ou le ruban adhésif transparent adapté aux applications cutanées (2) est composé(e) d'un adhésif à base d'acrylate, de silicone ou de caoutchouc.

3. Bandes fonctionnelles adhésives caractérisées selon la revendication 1 en ce que la (ou les) au moins une couche(s) adhésive(s) absorbant la lumière (4) est (ou sont) constituée(s) d'un adhésif pigmenté au noir de carbone et donc de couleur noire, à base d'acrylate, de silicone ou de caoutchouc.

4. Bandes fonctionnelles adhésives caractérisées selon les revendications 1 ou 2 en ce que la couche adhésive absorbant la lumière (4-6) consiste en une bande adhésive double face avec porteur pigmentée au noir de carbone et donc de couleur noire, à base d'acrylate, de silicone ou de caoutchouc (5).

5. Bandes fonctionnelles adhésives caractérisées selon la revendication 1 en ce que la bande adhésive double face absorbant la lumière comporte plusieurs encoches en forme de fenêtres (13) situés les unes à côté des autres et séparés par des nervures opaques (14), mettant les sources lumineuses, c'est-à-dire la tête du capteur avec lampe LED et la photodiode, directement en contact avec la peau,

6. Bande fonctionnelle adhésive caractérisée selon la revendication 1 en ce que les bandes d'étanchéité (8-11) sont constituées d'une mousse (9) dans les deux faces sont revêtues d'un adhésif (8, 10) à base d'acrylate, de silicone ou de caoutchouc.

7. Procédé de fabrication de bandes fonctionnelles adhésives caractérisé selon l'une des revendications ci-dessus en ce que la (ou les) encoches (13) est (ou sont) produite(s) lors d'un processus de poinçonnage.

8. Procédé de production de bandes fonctionnelles adhésives caractérisé selon l'une des revendications 1 à 6 ci-dessus en ce qu'il est produit dans une séquence d'opérations de laminage et de poinçonnage.

9. Utilisation d'une bande fonctionnelle adhésive caractérisée selon l'une des revendications 1 à 6 ci-dessus en ce qu'elle est réutilisable plusieurs fois ou peut être éliminée après une seule utilisation.

10. Utilisation d'une bande fonctionnelle adhésive caractérisée selon l'une des revendications 1 à 6 ci-dessus en ce qu'elle est utilisée pour la mesure transcutanée du taux de filtration glomérulaire (GFR).

11. Utilisation d'une bande fonctionnelle adhésive caractérisée selon l'une des revendications 1 à 6 ci-dessus en ce qu'elle peut être utilisée pour la mesure transcutanée des fonctions du foie, du cœur ou du pancréas ou encore pour la mesure des valeurs de lactate ou de glucose.
